# EUROPEAN PATENT SPECIFICATION

(11) **EP 1 539 156 B1**
(45) Date of publication and mention of the grant of the patent: **12.12.2012**
(21) Application number: 03763122.3
(22) Date of filing: 02.07.2003
(51) Int. Cl.: A61K 31/4439, A61K 9/08, A61K 9/10, A61K 9/58, A61K 9/26

(54) **LIQUID DOSAGE FORMS OF ACID LABILE DRUGS**
FLÜSSIGE DOSIERFORMEN SÄURELABILER ARZNEIMITTEL
FORMES POSOLOGIQUES LIQUIDES DE MEDICAMENTS LABILES ACIDES

(30) Priority: 03.07.2002 US 190242
(43) Date of publication of application: 15.06.2005
(73) Proprietor: ABBOTT LABORATORIES, Abbott Park IL 60064-3500 (US)
(72) Inventor: TANEJA, Rajneesh, Libertyville, IL 60048 (US)
(74) Representative: Modiano, Micaela Nadia
(86) International application number: PCT/US2003/020875
(87) International publication number: WO 2004/004718

(56) References cited:
- WO-A-00/74654
- WO-A-02/45692
- US-A- 4 786 505
- US-A- 5 464 632
- US-A- 5 731 002
- US-A- 6 132 770
- US-B1- 6 299 904
- US-B1- 6 328 994

## Description

### Technical Field

The present invention relates to liquid dosage forms and in particular, relates to liquid dosage forms of acid labile drugs.

### Background of the Invention

Many pharmaceutical compounds are susceptible to degradation in acidic environments. For example, antibiotics such as erythromycin; proton pump inhibitors (or "PPIs") such as Lansoprazole®, or Omeprazole®; and pencreatin; are compounds that degrade in acidic environments and are therefore referred to as "acid labile". Oral delivery of acid labile pharmaceutical compounds is challenging because the gastric pH is very acidic (typically between about pH 1.5 and 1.9). Under such conditions, acid-labile drugs typically degrade and are not readily available for uptake without being protected.

Due to the pH sensitivity of acid labile drugs, they typically are administered in a form that protects the drug from the acidic gastric environment. Enteric coatings are probably the most widely used method of protecting acid-labile drugs from gastric degradation. Enteric coating methods typically form a barrier around drug particles, or an entire dosage form containing an acid-labile drug, with a coating that does not dissolve upon introduction to the low pH of the gastric environment. Such enteric coatings typically dissolve at a pH greater than 6, such as that found in the upper small intestine where the acid labile drugs are released in an environment where they will not significantly degrade, and therefore can be absorbed. Unfortunately, enteric coated compositions are difficult to formulate as liquids, thus creating difficulty in administration to pediatric patients, patients having difficulty swallowing, or patients who cannot swallow at all such as when critically ill patients are fitted with a naso-gastric tube or a gastronomy tube (variously and collectively referred to as an "NG-tube") for feeding.

Notwithstanding the above, attempts have been made for formulate liquid dosage forms of acid-labile drugs. For example, U.S. Patent No. 5,840,737 recommends dissolving the contents of commercially available capsules containing enterically coated pellets of a PPI in a solution of sodium bicarbonate buffer. In practice, the above method requires a practitioner to open a capsule and release the enterically coated PPI into the buffer. After the contents of the capsule and buffer are combined, the mixture is swirled or mixed for approximately 20 to 30 minutes so that the enteric coating dissolves in the buffer's relatively high pH. Once the enteric coating is dissolved, the PPI is relatively stabile in the buffer and is able to be administered to a patient. However, the volume of buffer used in this practice is relatively large and can produce stomach gases and therefore belching which is detrimental to individuals suffering from gastro-esophageal reflux disease (GERD), one of the disease states a PPI is intended to alleviate. Additionally, the buffer employed typically is a separate component that adds to the cost of providing such a formulation. Additionally, when given orally, the taste of such a solution is unpleasant. Also, great care must be taken to completely dissolve the enteric coating layer from the enterically coated PPI since undissolved components of an enteric coating layer tend to form sticky globules that can stick together or stick to the walls of an NG-tube, for example.

There is therefore a need for a liquid dosage form for acid-labile drugs that maintains the efficacy of the active component, is easily administered to patients in a low volume of liquid, and is palatable.

### Summary of the Invention

In accordance with the present invention, liquid formulations of acid-labile drugs are provided that maintain the efficacy of the acid-labile drug, are easy to use and may be administered in relatively small volumes. The formulations generally comprise micro-granules comprising an acid-labile drug coated with an enteric coating and a liquid vehicle having a pH less than 6.0. The components of the formulation can be separately provided in the form of kits. The formulations and kits may be used to treat patients suffering from disorders for which the acid labile drugs are indicated.

### Detailed Description of the Invention

The present invention provides a liquid formulation of an acid labile drug that generally is simple to formulate and cost efficient. The formulation can be provided in relatively small volumes that are easily swallowed or administered through an NG-tube with little, if any, need for rinsing or flushing to provide a patient with a complete dose of an acid labile drug. As a result, patients are not provided with unnecessary liquids that, as mentioned above, can be contraindicated for certain patients. Additionally, the compositions provided herein are relatively quickly formulated.

Generally, the formulation comprises micro-granules of an enterically coated acid-labile drug and a liquid vehicle having a pH that will not dissolve the enteric coating, typically a pH of less than 6.0. The micro-granules typically will comprise an acid-labile drug protected by an enteric coat. Any acid-labile drug is suitable for use in the present invention and include, but are not limited to certain antibiotics such as erythromycin; proton pump inhibitors (or "PPIs") such as, for example, Lansoprazole®, omeprazole®, and pantoprazole; and pencreatin. Such drugs may be formulated with other active or inactive ingredients before being enterically coated. For example, stabilizers such as salts of group I or group II metals such as, for example, magnesium oxide, magnesium hydroxide, calcium carbonate, or sodium bicarbonate may be used in such formulations to maintain the integrity of the active drug; fillers such as talc; as well as sugars and other excipients such as sucrose, mannitol, and microcrystalline cellulose, may also be part of such formulations.

Micro-granules will also comprise a therapeutically effective amount of an acid-labile drug. A "therapeutically effective amount" as used herein means a sufficient amount of, for example, the composition, compound, or formulation necessary to treat the desired disorder, at a reasonable benefit/risk ratio applicable to any medical treatment. As with other pharmaceuticals, it will be understood that the total daily usage of a pharmaceutical composition of the invention will be decided by a patient's attending physician within the scope of sound medical judgment. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time administration, route of administration, and rate of excretion of the specific compound employed; the duration of the treatment; drugs used in combination or coincidental with the specific compound employed; and other factors known to those of ordinary skill in the medical arts. For example, it is well within the skill of the art to start doses of the compound at levels lower than required to achieve the desired therapeutic effect and to gradually increase the dosage until the desired effect is achieved.

Formulations of the invention are administered and dosed in accordance with sound medical practice, taking into account the clinical condition of the individual patient, the site and method of administration, scheduling of administration, and other factors known to medical practitioners.

Therapeutically effective amounts for purposes herein thus can readily be determined by such considerations as are known in the art. The amount must be affective to achieve improvement, including but not limited to, raising of gastric pH, reduced gastrointestinal bleeding, reduction in the need for blood transfusions, improved survival rate, more rapid recovery, and/or improvement/elimination of symptoms and other indicators as are selected as appropriate measures by those skilled in the art.

Those skilled in the art will recognize that the micro-granules may also comprise multiple coatings. Coatings, in addition to the enteric coat, may be applied to the core formulation prior to applying the enteric coating layer. Additional coatings typically are employed to protect the acid labile drug in cases where it may react with the enteric coating material. Hence, the additional coating(s) may be applied between the acid labile drug core and the enteric coat. Materials that typically are employed for this purpose include, but are not limited to hydroxy propyl cellulose and hydroxy propyl methyl cellulose (HPMC). The use of additional coating layers in a micro-granule is largely a matter of choice for those skilled in the art based upon the composition of the acid labile drug and enteric coating material employed. Such determinations are routinely made empirically by performing side-by-side stability studies on enterically coated micro-granules having sub-coatings and similarly composed micro-granules without sub-coatings.

Enteric coatings and methods for applying enteric coats are well known in the art. Enteric coatings generally comprise ingredients that do not dissolve in environments having a pH less than 6.0. Typically, enteric coatings of the present invention will protect the acid-labile drug in the pH of the liquid composition as well as the gastric environment. Any of the well known enteric coating materials are suitable for use with the present invention and may include polymers of compounds such as, for example, stearic acid, palmatic acid, and behenic acid; and polymers like hydroxy propyl methyl cellulose phthalate, polyvinyl acetate phthalate, cellulose acetate phthalate, methacrylic acid copolymers and cellulose acetate trimellitate.

Microgranules may take many different forms depending upon the granulation and sieving procedures employed but typically are, for the most part, spherical in nature and have a size range of between 100 µm and 900 µm, more preferably between 100 µm and 700 µm, and most preferably between 200 µm and 500 µm.

Liquid vehicles that may be employed according to the present invention may inherently have a pH of less than 6.0 or additional ingredients such as acidic excipients may be added to a liquid vehicle to achieve a pH of less than 6.0. Citric acid and ascorbic acid are are suitable for maintaining the pH of liquid below 6.0. Typically, the pH of the liquid vehicle is less than 6.0, preferably less than 5.5, more preferably less than 5.5, and most preferably less than 5.0.

Due to the nature of the present formulation the volume of the liquid vehicle employed can be extremely variable. While there is no upper limit to the volume of the liquid vehicle employed, practical considerations (as mentioned above) show the need for a low volume formulation. Advantageously, the present formulation can be administered in relatively low volumes. Typically, the volume of the liquid vehicle employed is less than 50 ml, preferably less than 25 ml and most preferably less than 10 ml. In most cases, the the volume of the liquid vehicle will be at least 0.5 ml, more preferably at least 2.0 ml, and most preferably at least 5.0 ml.

Many configurations for the compositions are possible. For example, the micro-granules can be formulated into a fast dissolving tablet such as those found in U.S. Patent Numbers 5,464,632 and 6,299,904 which are herein incorporated by reference. Such tablets may also contain an acidic excipient such that when the tablet is placed in water the micro-granules and acidic excipient are released. As a result, the acidic excipient produces a liquid having a pH of less than 6.0 that also contains the micro-granules. Alternatively, sachet formulations are also suitable for producing a composition comprising micro-granules and a liquid vehicle having a pH of less than 6.0. Sachets typically are packaged dry ingredients which, for present purposes, could contain enterically coated micro-granules of a acid labile drug and an acidic excipient. Similarly to a fast dissolving tablet, such a sachet formulation simply could be placed in small volume of water to form a composition comprising micro-granules and a liquid vehicle having a pH of less than 6.0. Of course, other ingredients such as flavoring agents, surfactants, sweetners, as well as other well known excipients could be added to such formulations.

The compositions of the present invention may be provided as kits containing separately packaged containers of the components of the composition. For example, a kit may contain a vial of water and a tablet containing dry ingredients of the formulation such as the enterically coated micro-granules and an acidic excipient. Upon transferring the tablet in the water provided, a liquid formulation is formed. It will be understood of course that the compositon may come as separate components, but the separate components should be mixed to form a liquid formulation comprising the microgranules in a liquid vehicle having a pH such as those specified above, prior to administration. In cases where kits are provided, the kits may come with instructions for appropriate mixing of the dry and liquid components. Additionally, it is preferable that the dry ingredients completely disperse in the liquid ingredients in less than 10 minutes, preferably in less than 5 minutes, and most preferably in less than 2 minutes.

Preferably, compositions of the present invention comprising a PPI are provided to patients experiencing gastrointestinal disorders such as, for example, acid reflux disease, gastro-esophogeal reflux disease, acid related gastrointestinal disorders such as peptic and duodenal ulcers, Zollinger Ellison Syndrome, or any other gastrointestinal disorder for which PPI's are indicated to thereby alleviate such disorders. The formulations are particularly well suited for patients outfitted with an NG-tube. It has been discovered that micro-granules flow freely and relatively completely through a patient's NG-tube without rinsing for purposes of insuring that the patient receives a complete dose of a prescribed medication. Hence, methods are provided for treating gastrointestinal disorders comprising administering a composition of the present invention comprising a PPI to a patient in need of such a therapy such as those experiencing gastrointestinal disorders.

### Examples

### Example 1

### Passage of Micro-granules Through Small Orifice

This experiment demonstrates that the Lansoprazole® micro-granules from a Lansoprazole® fast dissolving tablet ("LFDT"; TAP Pharmaceutical Products Inc., Lake Forest, IL) can pass through an orifice that is less than 2 mm in diameter. LFDT tablets contain enterically coated micro-granules of lansoprazole® that are about 350 microns in size. The tablets also comprise citric acid as an acidic excipient that is sufficient to lower the pH of 30 ml of water to less than 5.0.

An LFDT (30 mg) was placed in a 20 cc syringe from which the plunger had been removed. The plunger was replaced and the needle was uncapped. About 5 cc of sterile water for injection (SWFI) was drawn into the syringe. The syringe was gently shaken for about 45 seconds to ensure that the LFDT dissolved and formed a suspension. The contents of the syringe were injected into an empty flask. The syringe was examined for any residual contents. The contents of the flask were analyzed by HPLC. According to the HPLC analysis, greater than 95% of the drug was recovered in the flask, indicating that the micro-granules from LFDT freely passed through the orifice of the syringe.

### Example 2

### Stability of Micro-granule Compositions in Different Acidic Liquid Vehicles

This example demonstrates the stability of enterically coated microgranules in various liquid vehicles. The liquid vehicles employed in this experiment were SWFI and apple juice. After dissolution of LFDT tablets in the various liquid vehicles, the suspensions were added to simulated gastric fluid, or "SGF", containing 2.0 gm NaCl and 7 ml of HCl/1000ml of water. The ability of the micro-granules to protect the active ingredient Lansoprazole® was determined over time.

5.0 ml of SWFI was pulled into a 10 ml syringe containing a LFDT tablet. The syringe was tapped and gently swirled for approximately 45 seconds. The contents of the syringe were then delivered into a flask containing 50.0 ml of SGF. 5.0 ml of additional SWFI was pulled into the syringe and briefly swirled before the contents were placed in the flask containing the SGF. The flask was then allowed to sit for 30 minutes with gentle swirling at five minute intervals. 5.0 ml of 2N NaOH then was added to the flask after the 30 minute time period and the contents of the flask was diluted to 100 ml with pH 10 buffer (60:40:1 water:acetonitrile (ACN):triethylamine (TEA) pHed with H₃PO₄). The addition of the high pH buffer and NaOH increased the pH such that the enteric coating of the Lansoprazole® micro-granules dissolved and released the Lansoprazole® into the high pH buffer in which Lansoprazole® is stable. 5.0 ml of the solution from the flask was diluted with a further 25.0 ml of the pH 10 diluent. The diluted sample was then filtered through a 0.45um filter. The first 2.0 ml of filtrate was discarded and a 1.5 ml sample of the remaining filtrated was tested by HPLC for the presence of Lansoprazole®.

The procedure above was repeated except that the SWFI was replaced with apple juice having a pH of 3.9.

HPLC analysis on the above samples showed that after 30 minutes of suspension in SWFI or apple juice, 96.6% and 96.3% of the Lansoprazole® in the micro-granules respectively was recovered. Hence, the enteric coating on the mico-granules was stable in the acidic environments.

### Example 3

### Stability of Microgranules in Acidic Liquid Vehicle

This example demonstrates that micro-granules of Lansoprazole® (present in LFDT) are stable when LFDT is dissolved in water and held for 20, 30 and 60 minutes.

10.0 ml of SWFI was placed into each of three 30 ml beakers. A LFDT was placed into each of the beakers containing SWFI. After 20 minutes one beaker containing LFDT in SWFI was poured into a flask containing 490 ml of 0.1 N HCl warmed to 37°C. Prior to combining the contents of the beaker and flask, 10 ml of the 0.1 N HCl was removed from the flask and used to rinse the beaker containing the LFDT. Upon rinsing the beaker, the contents were returned to the flask. This procedure was repeated for the other beakers after 40 and 60 minutes.

After the contents of the beakers were added to the flasks containing the warm 0.1N HCl, the samples were allowed to incubate for 60 minutes at 37°C with constant stirring using a paddle on a motor set to rotate at 75 rpm. After the incubation times, 25 ml of the contents of the flasks were removed and 25 ml of 2N NaOH was then added to the flasks which increased the pH of the flasks to between 11 and 12 as determined with pH 0-14 pHing paper. Upon addition of the 2N NaOH, the rpm of the paddle was increased to 200 rpm and allowed to mix the samples for an additional 60 minutes. The addition of 2N NaOH dissolves the enteric coating and provides an environment where Lansoprazole® is stable. 10 ml aliquots of the flasks were then removed and filtered through a 45 µm filter. The first 2-3 ml of the filtrates was discarded and the remaining portion was used for HPLC analysis. HPLC was used to determine the amount of Lansoprazole® recovered from the final flasks. The percent of Lansoprazole® recovered is shown in Table 1 below over the time periods tested.

**Table 1**

| Time in SWFI, prior to 1hr. in 0.1N HCl (minutes) | % of lansoprazole® remaining **LFDT** |
|---|---|
| 20 | 92 |
| 40 | 90 |
| 60 | 88 |

As shown by Table 1, the LFDT formulation protected Lansoprazole® for extended periods of time when exposed to water prior to contact with acid

### Example 4

### Acidic Buffering Capacity of LFDT

This example was to designed to determine the effects of water volume on the pH of a solution containing the water and an LFDT.

One LFDT tablet was dispensed in to vials containing 1, 2, 3, 4, 5, 10, 20, and 30 ml of SWFI. The vials were gently swirled until the LFDT was completely disintegrated. The pH's of the vials was then measured 2-3 minutes after the LFDTs were completely disintegrated with an Accumet Model 915 pH meter equipped with pencil epoxy electrode. The disintegration time and pH of the various suspension is shown in Table 2 below.

**Table 2**

| Volume of SWFI (ml) | Time to disintegrate (seconds) | pH |
|---|---|---|
| 1 | 60 | 3.77 |
| 2 | 45 | 3.84 |
| 3 | 45 | 3.95 |
| 4 | 35 | 3.99 |
| 5 | 40 | 4.01 |
| 10 | 45 | 4.06 |
| 20 | 40 | 4.13 |
| 30 | 35 | 4.18 |

LFDT tablets can be dissolved in a wide variety of volumes of SWFI, while maintaining the pH of the resulting suspension below 4.5.

### Example 5

### NG-tube Administration of Liquid Dosage Forms

This experiment analyzed the volume of liquid used to pass micro-granules from LFDT through a NG-tube.

An LFDT tablet was dispensed into two separate 35 ml catheter-tipped syringes. 10 ml of apple juice was added to one syringe and 10 ml of distilled water was added to the other syringe and both syringes were shaken. The contents of the syringes were then delivered to separate NG-tubes that were configured in a manner designed to mimic the curvation and length of a NG-tube in an actual patient. The contents of the liquid flowing through the NG-tube were collected in a flask attached at the opposite end of the NG-tube. The syringes were then rinsed once with 10 ml of the suspension liquid (i.e. either apple juice or distilled water). The rinse fluid was transferred to the NG-tube and collected in the flask as above. Accordingly, a total of 20 ml of liquid was transferred through the NG-tube.

The 20 ml samples collected from the NG tubes were combined with 1.0 ml of 2N NaOH and mixed well before an additional 30 ml of 0.1N NaOH were added to the samples. The contents of the flasks were then diluted to 100 ml with ACN and mixed well. 10.0 ml of this solution was then diluted to 50.0 ml with pH 10 diluent (2400 ml HPLC grade water, 1600 ml of HPLC grade ACN, 40 ml HPLC grade TEA, pHed to 10.0 with H3PO4). A portion of this solution was then filtered through a 0.45 µm filter. The first 2 ml of filtrate was discarded and the next 1.5 ml was collected for HPLC analysis to determine the amount of Lansoprazole® recovered at the end of the NG-tube. The results of the HPLC analysis is shown in Table 3 below.

**Table 3**

| **Test Sample** | **Gastrostomy tube size** | **Total volume of suspending vehicle** | **% Lansoprazole® recovered through tube using Distilled Water** | **% Lansoprazole® recovered through tube using Apple Juice** |
|---|---|---|---|---|
| LFDT tablets | **12 F** | 20 mL | 100.1 | 97.9 |

As shown in Table 3, 100.1% and 97.9 % of Lansoprazole® was recovered when the LFDT micro-granules were administered through a 12 F NG-tube utilizing distilled water and apple juice, respectively. These results demonstrate the benefit of administering micro-granules via a gastrostomy tube.

## Claims

1. A liquid pharmaceutical formulation comprising:
(a) micro-granules comprising an enterically coated proton pump inhibitor, and
(b) a liquid vehicle having a pH less than 6.0.

2. The composition of claim 1 wherein the micro-granules are between 100 µm and 900 µm.

3. The composition of claim 1 wherein the proton pump inhibitor is Lansoprazole.

4. The composition of claim 1 wherein the liquid has a volume less than 50 ml.

5. A liquid formulation for use for treating a patient with a gastrointestinal disorder comprising micro-granules of an enterically coated proton pump inhibitor and a liquid vehicle having a pH less than 6.0.

6. A kit for a liquid formulation according to claim 1 comprising:
a) a first container comprising micro-granules comprising an enterically coated proton pump inhibitor; and
b) a second container comprising a liquid vehicle;
wherein the first or second container further comprises an acidic excipient.

7. The kit of claim 6 wherein the proton pump inhibitor is Lansoprazole.

8. The kit of claim 6 wherein the micro-granules are between 100 µm and 900 µm.

9. The kit of claim 6 wherein the volume of the liquid vehicle in the second container is less than 50 ml.

## Patentansprüche

1. Eine flüssige pharmazeutische Formulierung, die Folgendes umfasst:
(a) Mikrokörnchen, die einen enterisch beschichteten Protonenpumpenhemmer umfassen, und
(b) ein flüssiges Bindemittel mit einem pH von weniger als 6,0.

2. Die Zusammensetzung gemäß Anspruch 1, worin die Mikrokörnchen zwischen 100 µm und 900 µm sind.

3. Die Zusammensetzung gemäß Anspruch 1, worin der Protonenpumpenhemmer Lansoprazol ist.

4. Die Zusammensetzung gemäß Anspruch 1, worin die Flüssigkeit ein Volumen von weniger als 50 ml hat.

5. Eine flüssige Formulierung zur Verwendung für die Behandlung eines Patienten mit einer gastrointestinalen Erkrankung, umfassend Mikrokörnchen eines enterisch beschichteten Protonenpumpenhemmers und eines flüssigen Bindemittels mit einem pH von weniger als 6,0.

6. Ein Kit für eine flüssige Formulierung gemäß Anspruch 1, das Folgendes umfasst:
a) einen ersten Behälter, der Mikrokörnchen umfasst, welche einen enterisch beschichteten Protonenpumpenhemmer umfassen, und
b) einen zweiten Behälter, der ein flüssiges Bindemittel umfasst,
worin der erste oder zweite Behälter weiter ein saures Bindemittel umfasst.

7. Das Kit gemäß Anspruch 6, worin der Protonenpumpenhemmer Lansoprazol ist.

8. Das Kit gemäß Anspruch 6, worin die Mikrokörnchen zwischen 100 pm und 900 µm sind.

9. Das Kit gemäß Anspruch 6, worin das Volumen des flüssigen Bindemittels im zweiten Behälter weniger als 50 ml beträgt.

## Revendications

1. Formulation pharmaceutique liquide comprenant :
(a) des micro-granules comprenant un inhibiteur de la pompe à protons enrobé entériquement ; et
(b) un véhicule liquide ayant un pH inférieur à 6,0.

2. Composition selon la revendication 1, dans laquelle les micro-granules sont entre 100 µm et 900 µm.

3. Composition selon la revendication 1, dans laquelle l'inhibiteur de la pompe à protons est le Lansoprazole.

4. Composition selon la revendication 1, dans laquelle le liquide a un volume inférieur à 50 ml.

5. Formulation liquide pour une utilisation pour traiter un patient ayant un trouble gastro-intestinal comprenant des micro-granules d'un inhibiteur de la pompe à protons enrobé entériquement et un véhicule liquide ayant un pH inférieur à 6,0.

6. Kit pour une formulation liquide selon la revendication 1, comprenant :
a) un premier contenant comprenant des micro-granules comprenant un inhibiteur de la pompe à protons enrobé entériquement ; et
b) un second contenant comprenant un véhicule liquide ;
dans lequel le premier ou le second contenant comprend en outre un excipient acide.

7. Kit selon la revendication 6, dans lequel l'inhibiteur de la pompe à protons est le Lansoprazole.

8. Kit selon la revendication 6, dans lequel les micro-granules sont entre 100 µm et 900 µm.

9. Kit selon la revendication 6, dans lequel le volume du véhicule liquide dans le second contenant est inférieur à 50 ml.
